Europäisches Patentamt

European Patent Office

Office européen des brevets

(11) Veröffentlichungsnummer: **0 355 254**
**A1**

## EUROPÄISCHE PATENTANMELDUNG

(21) Anmeldenummer: **89104782.1**

(22) Anmeldetag: **26.04.89**

(51) Int. Cl.4: **A61H 33/04**

(30) Priorität: **20.08.88 DE 3829490**

(43) Veröffentlichungstag der Anmeldung:
**28.02.90 Patentblatt 90/09**

(84) Benannte Vertragsstaaten:
**AT BE CH DE ES FR GB GR IT LI LU NL SE**

(71) Anmelder: **Smrz, Peter, Prof. Dr. med.**
**Neue Strasse 125**
**D-7900 Ulm(DE)**

(72) Erfinder: **Smrz, Peter, Prof. Dr. med.**
**Neue Strasse 125**
**D-7900 Ulm(DE)**

(54) **Imprägnieren von Textilien mit Meersalz zu therapeutischen Zwecken.**

(57) Um eine genügend hohe Salzkonzentration zu therapeutischen Zwecken zu erzielen, wird ein Baumwollhemd, -Strümpfe, -Hose mit einer Wasser-Salzlösung getränkt und auf der Haut getragen.

EP 0 355 254 A1

EP 0 355 254 A1

## Verfahren zum Aufbringen von Meersalz auf die Haut zu Therapie-Zwecken

Der Erfindung liegt die Aufgabe zugrunde, eine hohe Salzkonzentration auf der nassen Haut zu erzielen. Dadurch ergibt sich eine Reiseersparnis zum Beispiel für Psoriatiker nach Israel zum Toten Meer. Um eine ähnlich hohe Salzkonzentration in der Badewanne zu erreichen, wäre etwa die 150fache Menge an Salz erforderlich, somit kommt es bei dem oben beschriebenen Verfahren zu einer wesentlichen Kostenersparnis.

**Ansprüche**

1. Zur Anwendung kommt Meersalz, welches mit einem Farbstoff zur psychologischen Wirkungssteigerung versetzt mird.

2. Das Salzgemisch (150 g) wird in 1 Liter Wasser aufgelöst. In dieser Salzlösung wird ein Baumwollhemd, -Hose, -Strümpfe (100 % Baumwolle) 5 Minuten liegen gelassen.

Das nun salzgetränkte Hemd wird naß angezogen, und der Patient wird nun in eine Folie und darüber in eine Decke gewickelt. Nach 20 Minuten wird der Körper mit Wasser abgeduscht und die Prozedur ist beendet.

3. <u>Zusammensetzung:</u>

1 Portion Salzmischung enthält:

150 g Meersalz

| | | | |
|---|---|---|---|
| Natriumchlorid | NaCl | $\sim$ | 99,9 % |
| Calcium | Ca | | 0,03 % |
| Magnesium | Mg | $<$ | 0,01 % |
| Sulfat | $SO_4$ | | 0,07 % |
| Unlösliches | | | ---- |
| Blei | Pb | | 0,13 % |

0,75 g im Handel erhältlichen Lebensmittelfarbstoff

blau = E 132 Indigotin

4. Der Erfindung liegt die Aufgabe zugrunde, eine hohe Salzkonzentrication auf der nasse Haut zu erzielen. Dadurch ergibt sich eine Reiseersparnis zum Beispiel für Psoriatiker nach Israel zum Toten Meer. Um eine ähnlich hohe Salzkonzentration in der Badewanne zu erreichen, wäre etwa die 150fache Menge an Salz erforderlich, somit kommt es bei dem oben beschriebenen Verfahren zu einer wesentlichen Kostenersparnis.

2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS Band 107, Nr. 25, 21. Dezember 1987, Seite 83, Spalte 1, Zusammenfassung Nr. 229115s, Columbus, Ohio, USA; J. SHANI et al.: "Effect of Dead Sea brine and its main salts on cell growth in culture" & Pharmacology 1987, Band 35, Nr. 6, Seiten 339-347 * Zusammenfassung * <br>--- | 1-4 | A 61 H 33/04 |
| X | EMBASE Zusammenfassung Nr. 75174231, 1975; W.W. AVRACH et al.: "Treatment of psoriasis at the Dead Sea" & Ugeskr.Laeg. 1974, Band 136, Nr. 48, Seiten 2687-2690 <br>--- | 1-4 | |
| X | EMBASE Zusammenfassung Nr. 85168086, 1985; J. SHANI et al.: "Skin penetration of minerals in psoriatics and guinea-pigs bathing in hypertonic salt solution" & Pharm.Res.Commun. 1985, Band 17, Nr. 6, Seiten 501-512 <br>--- | 1-4 | RECHERCHIERTE SACHGEBIETE (Int. Cl.5) |
| A | FR-A-1 590 474 (R. GEHRINGER) * das ganze Dokument * <br>--- | 1-4 | A 61 K<br>A 61 L<br>A 61 H |
| A | FR-A-2 590 270 (C. BREVIER) * das ganze Dokument * <br>---      -/- | 1-4 | |

Der vorliegende Recherchenbericht wurde für alle Patentansprüche erstellt

| Recherchenort | Abschlußdatum der Recherche | Prüfer |
|---|---|---|
| BERLIN | 24-10-1989 | AVEDIKIAN P.F. |

EPO FORM 1503 03.82 (P0403)

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EMBASE Zusammenfassung Nr. 82188726, 1982; J. BOER et al.: "Influence of water and salt solution on UVB irradiation of normal skin and psoriasis" & ARCH.DERMATOL.RES. 1982, Band 273, Nr. 3-4, Seiten 247-259 ----- | 1-4 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| BERLIN | 24-10-1989 | AVEDIKIAN P.F. |

EPO FORM 1503 03.82 (P0403)